# EUROPÄISCHE PATENTSCHRIFT

(11) **EP 1 563 311 B1**
(45) Veröffentlichungstag und Bekanntmachung des Hinweises auf die Patenterteilung: **03.01.2007**
(21) Anmeldenummer: 03758084.2
(22) Anmeldetag: 30.10.2003
(51) Int. Cl.: G01N 33/94

(54) **MARKERSUBSTANZEN UND IHRE VERWENDUNG IN DIAGNOSEVERFAHREN**
MARKER SUBSTANCES AND THE USE OF THE SAME IN DIAGNOSTIC METHODS
SUBSTANCES DE MARQUAGE ET LEUR UTILISATION DANS DES PROCEDES DE DIAGNOSTIC

(30) Priorität: 18.11.2002 DE 10253664
(43) Veröffentlichungstag der Anmeldung: 17.08.2005
(73) Patentinhaber: Gauchel, Gisela, 53332 Bornheim (DE)
(72) Erfinder: Gauchel, Gisela, 53332 Bornheim (DE)
(74) Vertreter: Zeitler, Giselher
(86) Internationale Anmeldenummer: PCT/EP2003/012079
(87) Internationale Veröffentlichungsnummer: WO 2004/046715

(56) Entgegenhaltungen:
- WO-A-98/12557
- WO-A-02/075307
- GB-A- 2 246 707
- US-A- 5 179 027
- US-A- 6 068 981
- DATABASE WPI Section Ch, Week 199428 Derwent Publications Ltd., London, GB; Class A96, AN 1994-235055 XP002268770 & ZA 9 306 383 A (HAK B W) 25. Mai 1994 (1994-05-25)
- DATABASE BIOSIS [Online] BIOSCIENCES INFORMATION SERVICE, PHILADELPHIA, PA, US; 1983, HINDMARSH K W ET AL: "URINARY EXCRETION OF METHYLPARABEN AND ITS METABOLITES IN PRETERM INFANTS" XP002267418 Database accession no. PREV198477048079 & JOURNAL OF PHARMACEUTICAL SCIENCES, Bd. 72, Nr. 9, 1983, Seiten 1039-1041, ISSN: 0022-3549

## Beschreibung

Die Erfindung betrifft ein in vitro Diagnoseverfahren, angewendet auf eine Urinprobe eines Probanden zur Ermittlung missbräuchlicher Manipulation der zu diagnostizierenden Probe durch den Probanden gemäß dem Oberbegriff des Anspruchs 1.

Zur Therapie drogensüchtiger Patienten sind die sog. Methadon- und Heroinprogramme eingeführt worden. Den an ihnen teilnehmenden Probanden ist der Konsum anderer illegaler Drogen bzw. Betäubungsmittel untersagt, strenge Kontrollen des sog. Beigebrauchs im Urin sind gesetzlich vorgeschrieben. Vor allem der Beigebrauch von Opiaten und Kokain muss zur Abschätzung des Substitutionsbedarfes bekannt sein [S.T. Chermack et al., Drug Alcohol Depend 59 (2000) 43-49]. Ständiger Beigebrauch gefährdet das Behandlungsziel der völligen Drogenabstinenz, welches Vermeidung akuter Entzugssymptomatik, Verhinderung opiatassoziierter Todesfälle, Reduzierung des Infektionsrisikos mit HI- und Hepatitisviren, Schutz der Bevölkerung vor Beschaffungskriminalität, Resozialisierung und berufliche Reintegration beinhaltet. Vor allem wegen der steigenden Zahl methadonassozüerter Todesfälle [Die Beauftragte der Bundesregierung. Sucht-und Drogenbericht 2001. Bundesministerium für Familie und Gesundheit, Berlin, April 2002] kann nachgewiesener häufiger Beigebrauch zum gesetzlich vorgeschriebenen Ausschluss aus dem jeweiligen Substitutionsprogramm führen [Bundesausschuß der Ärzte und Krankenkassen: Richtlinien des Bundesausschusses der Ärzte und Krankenkassen zur substitutionsgestützten Behandlung Opiatabhängiger. Köln, 1999]. Daher besteht seitens der Drogensüchtigen ein großes Interesse, falsch - negative Resultate bei der Drogenanalytik im Urin zu erzielen. Üblicherweise lässt sich das durch exzessives Trinken zwecks starker Verdünnung des Urins, durch Zumischung präanalytischer Störsubstanzen mit hohem Verfälschungspotential [S.L. Mikkelsen et al., Clin. Chem. 34 (1988) 2333 - 2336] oder durch Austausch des eigenen Urins gegen einen käuflich erwerbbaren methadonhaltigen aber drogenfreien Urin erreichen. Jedes neue Drogennachweisverfahren wird sofort durch Gegenmaßnahmen aus der Drogenszene konterkariert. Der Wettstreit zwischen Szene und Analytik lässt sich durch einschlägige Publikationen in Zeitschriften wie "High Times" belegen und verfolgen. Eine Aufgabe der Drogenanalytik besteht darin, diese mit Ideenreichtum und hohem Wissenstand durchgeführten Verfälschungen und Täuschungsmanöver zu erkennen und möglicherweise unwirksam zu machen.

DE 101 12 470 A1 beschreibt ein Verfahren der endogenen Markierung von Säugetieren, das die eindeutige Zuordnung einer Körperflüssigkeit, eines Sekretes, einer Gewebeprobe oder eines Exkrementes zu einem Individuum ermöglichen soll. Es besteht in der oralen oder parenteralen Gabe von nur geringfügig bzw. nicht metabolisierbaren Substanzen und deren anschließenden analytischen Nachweis als solche oder in Form ihrer Metaboliten in oben genannten Probenmaterialien. Dieses Konzept hat sich bereits in der klinischen Routine mit Polyethylenglykol (PEG) - Markern zur Überwachung drogenabhängiger Probanden unter Methadonsubstitutionstherapie gut bewährt. Die Probanden trinken 100 ml der mit Markersubstanzen versetzten Methadontrinklösung und liefern nach einer Wartezeit von 30 bis 60 Minuten ihren Urin ab. Dieser wird zentrifugiert und mittels Hochleistungsflüssigkeitschromatographie auf Nucleosil C18, 3µm, 125 mm x 4,6 mm ID mit einem Methanol - Wasser Gemisch (44% / 56%) bei einer Fließgeschwindigkeit von 0.5 ml/min analysiert. Die Probenvorbereitung erfolgt automatisch online mittels column - switching auf Nucleosil C18 5µm, 60 mm x 4.6 mm ID, die Charakterisierung der Marker im Eluat mittels Brechungsindex - Detektion (RID).

Auch dieses Verfahren der endogenen Markierung ist manipulationsanfällig. Es wird von den Probanden der Methadonambulanzen unwirksam gemacht, indem diese Reste der Trinklösung in drogenfreien Urin spucken bzw. einen im Mund mit Trinklösung getränkten Wattebausch in beigebrauchsfreien Urin ausdrücken.

Es ist weiterhin ein Verfahren bekannt (US 5 179 027), bei dem dem Probanden verschiedene Markersubstanzen zugeführt werden, die beide im Urin nachweisbar sein sollen. In aufwändigen, geschultes Personal erfordernden Diagnosen sollen quantitativ die beiden Markersubstanzen hinsichtlich der Menge und des Verhältnisses zueinander festgestellt werden. Ein zeitlicher Rahmen nur für die Abgabe des Sekretes ist mit 24 h angegeben. Darüber hinaus sollen die Dosierungen und Kombinationen für die einzelnen Individuen variiert und mit den Dosierungszeiten dokumentiert werden. Nach Beendigung der Sammelperiode werden Sammelmenge und -zeit registriert.

Als Merker für die Zuordnung einer Probe zu einem Individuum wird die nicht metabolisierbare körpereigene Substanz Kreatinin eingesetzt, die zur Unterscheidung von körpereigenem Kreatinin und zur Erhöhung der Anzahl der Marker mit unterschiedlichen nicht-radioaktiven stabilen Isotopen gelabelt ist. Für die Ingestion dieser Isotope sind nur Spuren erforderlich, um die untere Nachweisgrenze de GC/MS Analyse zu überschreiten. Die Prüfung auf Vollständigkeit einer Probe erfolgt durch die quantitative Bestimmung der teilweise metabolisierbaren Markersubstanz PABA (P-amino-benzoesäure), seiner Steroisomeren, deren Salze und Metaboliten.

Bei der Anwendung dieses bekannten Verfahrens wird die Kontamination eines Fremdurins mit Marker-Trinklösung erkennbar durch die Bestimmung des PABA und seiner Abkömmlinge.

Hier setzt die Erfindung ein. Es liegt dementsprechend der Erfindung die Aufgabe zugrunde, ein in vitro-Diagnoseverfahren bereitzustellen, durch welches sich Manipulationen im Rahmen der endogenen Markierung aufdecken lassen. Die hierfür verwendeten Markersubstanzen sollen vorteilhafterweise keine zusätzliche Belastung der Probanden darstellen und darüber hinaus keine zusätzlichen Material- und Personalkosten verursachen.

Diese Aufgabe wird durch die Erfindung mit dem in vitro-Diagnoseverfahren gemäß Anspruch 1 gelöst. Eine vorteilhafte Ausgestaltung hiervon ist in Anspruch 2 angegeben.

Bei dem erfindungsgemäßen in vitro-Diagnoseverfahren wird dem Probanden außer den nur geringfügig oder nicht metabolisierbaren PEG-Markersubstanzen gleichzeitig und zusätzlich eine metabolisierbare Substanz aus der Gruppe der Benzoesäure-4-Hydroxybenzoesäurederivate zugeführt, die mit den nicht metabolisierbaren PEG-Markersubstanzen in gleichen Chromatogramm nachgewiesen kann und die nach dem Metabolismus In der Probe nicht mehr nachweisbar ist.

Die dem Probanden zusätzlich zugeführten Markersubstanzen metabolisieren im Körper, so dass sie im Urin nicht mehr vorhanden sind. Bei einem Versuch des Probanden, durch Spucken in den Urin die Zuordnung zu manipulieren, lässt sich die Markersubstanz dort nachweisen, so dass sich eine Manipulation sicher verhindern lässt. Aus diesem Grunde ist der Term "Spuckmarker" eine treffende Charakterisierung.

Wenn also der Trinklösung metabolisierbare Substanzen als Spuckmarker zugemischt werden, die üblicherweise in Urinen nicht vorhanden sind, lässt deren Nachweis dort ein Täuschungsmanöver sicher erkennen.

Zwar erwähnt die DE 101 12 470 A1, dass Proben aus Speichel genommen werden können. Aber die dort beschriebene Probenahme dient der Identfizierung illegal aufgenommener Substanzen sowie der Zuordnung der Probe zu einem Individuum und kann nicht den Nachweis auf Täuschungsabsichten hinsichtlich der Probenzuordnung erbringen.

Ideale Spuckmarker sind metabolisierbare Substanzen, die als Lebensmittel- und Arzneimittelzusätze zugelassenen sind. Sie sind nach oraler Aufnahme normalerweise nicht in intakter Form im Urin vorhanden. Eine Verfälschungsabsicht bezüglich des Analysenergebnisses liegt vor, wenn die als "Spuckmarker" eingesetzte Substanz in der Markeranalyse im Urin nachgewiesen wird.

Die erfindungsgemäßen Spuckmarkersubstanzen sind in der Trinklösung leicht löslich. Sie besitzen bei den erforderlichen Konzentrationen keine pharmakologische Wirkung, keine auffällige Farbe und keinen intensiven Geschmack. Sie verhalten sich chromatographisch ähnlich wie die verwendeten PEG - Markersubstanzen.

Die erfindungsgemäßen Spuckmarkersubstanzen sind durch die in klinisch chemischen Laboratorien etablierten Analysen- und Detektionsverfahren leicht nachweisbar.

Die Spuckmarkersubstanzen der Erfindung sind in Ansprüchen 1 und 2 definiert.

Bevorzugte Markersubstanzen sind die als Konservierungsstoffe zugelassenen sog. Nipa - Ester Methyl-, Ethyl-, und Propyl-4-hydroxybenzoat. Die Methadontrinklösung muss konserviert werden, und wenn die o.g. Konservierungsmittel benutzt werden, kommt es zu keiner zusätzlichen Belastung der Probanden und keinen zusätzlichen Kosten. Von den o.g. Konservierungsmitteln ist Methyl - 4 - hydroxybenzoat (MHB) am geeignetsten, da die chromatographischen Eigenschaften dieser Substanz sehr ähnlich denen der PEG - Marker sind. Es wird zusammen mit den PEG - Markern von der Säule eluiert, zusätzliche Analysenschritte sind nicht erforderlich.

Methyl-4-hydroxybenzoat, auch als Methylparaben und Nipagin-M^{®} bezeichnet, ist unter der Nummer E 218 als Lebensmittel- und Arzneimittelzusatzstoff zugelassen (Rote Liste). Es wird vor allem zur Konservierung pharmazeutischer Zubereitungen wie Augentropfen, Salben und Emulsionen verwendet und ist wegen seiner geringen Toxizität zur Konservierung von Lebensmitteln zugelassen.

Neben der Anwendung in Methadonambulanzen können "Spuckmarker" im Rahmen der endogenen Markierung in allen Bereichen der Human- und Veterinärmedizin eingesetzt werden, in denen ein Interesse besteht, die Herkunft von Proben in betrügerischer Absicht verschleiern zu wollen und in denen die Möglichkeit besteht, Proben mit der Markersubstanz zu kontaminieren. Alle in der DE 101 12 470 A1 erwähnten täuschungsbereiten Kollektive sind auf diese Weise kontrollierbar. Beispiele sind u.a. Dopingkontrolle im Leistungssport und im Tierwettkampf, Überwachung des Straßenverkehrs und des Personentransportes, Überwachung der anhaltenden Drogenfreiheit zur Wiedererlangung der Fahrerlaubnis, Einstellungsuntersuchungen (Reagan Erlass), personalärztliche Untersuchungen und veterinärmedizinische Kontrollen.

Die Analyse der Spuckmarker erfolgt im Rahmen der endogenen Markierung zusammen mit den PEG - Markern mittels Hochleistungsflüssigkeitschromatographie und anschließender RI - Detektion. Methyl - 4 - hydroxybenzoat besitzt nur einen kleinen Brechungsindex, aber einen ausreichend guten Absorptionskoeffizienten bei 256 nm. Wird ein UV - Detektor in Serie mit dem RI - Detektor geschaltet, kann die Belastung der Probanden um einen Faktor >10 vermindert werden. Alle Markersignale im Chromatogramm werden über die aus Standard- und Referenzchromatogrammen ermittelten Retentionszeiten ausgewertet.

### Beispiel

An einem Beispiel soll erklärt werden, wie, wenn die endogene Markierung zur Anwendung kommt, Täuschungsmanöver verhindert werden.

Einem Probanden werden 100 ml Methadontrinklösung verabreicht, die 1 - 3 g PEG - Markergemisch enthält und mit 0.01 % (10 mg/100 ml) Methyl - 4- hydroxybenzoat konserviert ist. Die Analyse wird entsprechend der in DE 101 12 470 A1 beschriebenen Vorschrift durchgeführt. Nach 30 - 60 Minuten liefert der Proband seinen Urin ab. Zusätzlich wird das Eluat nach der Passage des RI - Detektors von einem in Serie geschalteten UV - Detektor bei λ = 256 nm gemessen. Es entsteht ein Chromatogramm, in dem das charakteristische RID - Profil der verabreichten PEG - Marker mit der Aufzeichnung des UV - Detektors überlagert ist.

Zeichnet der UV - Detektor nur eine Basislinie im Retentionsbereich des Spuckmarkers auf und identifiziert der RI - Detektor die verabreichte Markerzusammensetzung, kann die Urinprobe eindeutig dem betreffenden Probanden zugeordnet werden. Enthält das UV - Chromatogramm dagegen zur Retentionszeit des Spuckmarkers einen Peak, kann davon ausgegangen werden, dass der Proband mit vorsätzlicher Täuschungsabsicht beigebrauchsfreien Urin mit Markersubstanz versetzt hat.

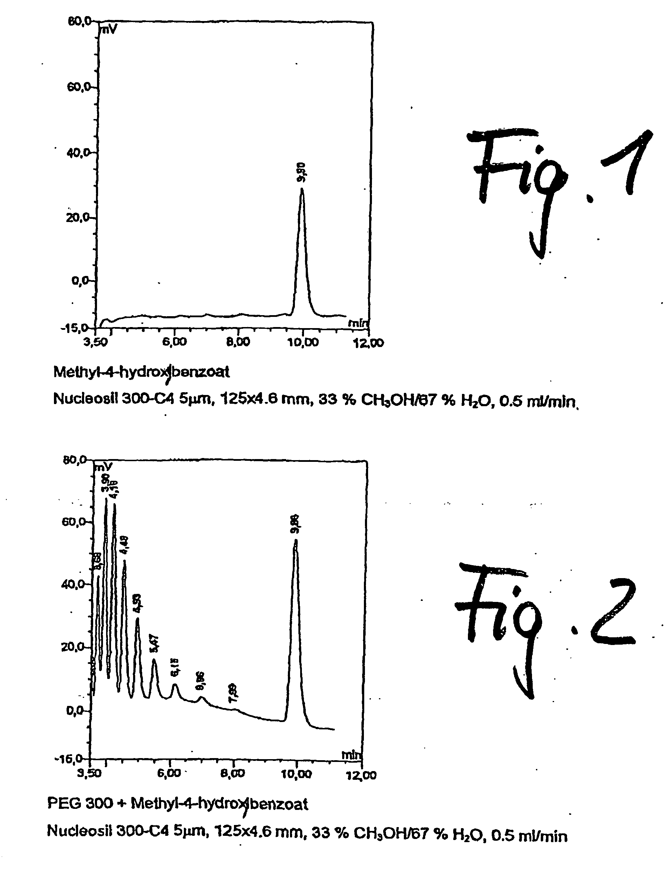

Beigefügt sind zwei Chromatogramme gemäß Fig. 1 und 2, und zwar eines für den Spuckmarker Methyl - 4 - hydroxybenzoat allein (Fig. 1) und ein zweites gemeinsam mit der Markersubstanz PEG 300 (Fig. 2).

Wenn monodisperse PEG - Fraktionen als Markersubstanzen zum Einsatz kommen, ist es hinsichtlich der Analysengeschwindigkeit vorteilhaft, das chromatographische System zu ändern. Werden kürzerkettige PEGs mit kürzeren Permeationszeiten bevorzugt, sollte die Analyse auf Nucleosil 300-C4 5µm, 125 mm x 4.6 mm ID mit 33 % CH₃OH / 67 % H₂O durchgeführt werden. Andernfalls führt die längere Verweilzeit des MHB auf der stationären Phase zu verlängerten Analysenzeiten.

## Patentansprüche

1. In vitro Diagnoseverfahren angewendet auf eine Urinprobe eines Probanden zur Ermittlung missbräuchlicher Manipulation der zu diagnostizierenden Probe durch den Probanden, bei welchem dem Probanden die nur geringfügig oder nicht metabolisierbaren Markersubstanzen PEG (Polyethylenglykole) zugeführt wurden, die in Urin diagnostizierbar sind,
**dadurch gekennzeichnet,**
**dass** dem Probanden außer den vorgenannten Markersubstanzen gleichzeitig und zusätzlich eine metabolisierbare Substanz aus der Gruppe der Benzoesäure- und 4-Hydroxybenzoesäurederivate zugeführt wurde, die mit den nicht metabolisierbaren PEG-Markersubstanzen im gleichen Chromatogramm nachgewiesen werden kann und die nach dem Metabolismus nicht mehr in der Probe nachweisbar ist, in der die Markersubstanzen analysiert werden.

2. Verfahren nach Anspruch 1, **dadurch gekennzeichnet, dass** als metabolisierbare Substanz Methyl - 4 - hydroxybenzoat verwendet wird.

## Claims

1. In vitro method of diagnosis, applied to a urine sample from a test subject for the determination of improper manipulation by the test subject of the sample to be diagnosed, in which the marker substances PEG (polyethylene glycols), which are only slightly metabolisable or are non-metabolisable and are diagnosable in urine, were administered to the test subject, **characterised in that**, as well as the above-mentioned marker substances, there was administered to the test subject, at the same time and in addition, a metabolisable substance from the group comprising derivates of benzoic acid and 4-hydroxybenzoic acid, which can be detected in the same chromatogram with the non-metabolisable PEG marker substances and which, after metabolism, are no longer detectable in the sample in which the marker substances are analysed.

2. Method according to claim 1, **characterised in that** methyl-4-hydroxybenzoate is used as a metabolisable substance.

## Revendications

1. Procédé de diagnostic in vitro, appliqué à un échantillon d'urine d'un sujet à tester pour constater une manipulation abusive de l'échantillon à diagnostiquer par le sujet à tester, dans lequel on administre au sujet à tester les substances de marquage PEG (polyéthylène-glycol) qui ne peuvent pas être métabolisées ou qui ne peuvent l'être que faiblement, et qui peuvent être diagnostiquées dans l'urine,
**caractérisé en ce que**
on administre au sujet à tester, outre les substances de marquage précitées, simultanément et en supplément une substance capable d'être métabolisée, choisie dans le groupe des dérivés de l'acide benzoïque et de l'acide 4-hydroxy-benzoïque, qui peut être décelée avec les substances de marquage PEG qui ne peuvent pas être métabolisées, dans le même chromatogramme, et qui, après le métabolisme, ne peut plus être décelée dans l'échantillon dans lequel les substances de marquage sont analysées.

2. Procédé selon la revendication 1, **caractérisé en ce que** l'on utilise à titre de substance capable d'être métabolisée du méthyle-4-hydroxybenzoate.
